(19) Europäisches Patentamt
European Patent Office
Office européen des brevets

(11) Publication number : **0 478 235 A2**

# (12) EUROPEAN PATENT APPLICATION

(21) Application number : **91308586.6**

(22) Date of filing : **20.09.91**

(51) Int. Cl.$^5$ : **C12P 17/18,** C07D 498/18, // (C12P17/18, C12R1:55)

The applicant has filed a statement in accordance with Rule 28 (4) EPC (issue of a sample only to an expert). Accession number(s) of the deposit(s): ATCC 55087.

(30) Priority : **24.09.90 US 587187**

(43) Date of publication of application : **01.04.92 Bulletin 92/14**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Byrne, Kevin, M.**
**6 Forest Lane**
**West Trenton, NJ 08628 (US)**
Inventor : **Arison, Byron H.**
**88 Century Lane**
**Watchung, NJ 07060 (US)**
Inventor : **Kaplan, Louis**
**7 Lexington Road**
**New City, NY 10956 (US)**
Inventor : **Dumont, Francis**
**54 West Cherry Street**
**Rahway NJ 07065 (US)**
Inventor : **Kahn, Jennifer N.**
**7 Elizabeth Avenue**
**East Brunswick, NJ 08816 (US)**

(74) Representative : **Thompson, John Dr. et al**
**Merck & Co., Inc. European Patent Department Terlings Park Eastwick Road**
**Harlow, Essex CM20 2QR (GB)**

## (54) A directed biosynthesis for an immunomycin derivative.

(57) This invention relates to a novel process for producing a new proline derivative of immunomycin (FK-520), designated "prolyl-immunomycin" that has immunosuppressant activity. This process involves directed biosynthesis, a fermentation process in which a cultured Streptomyces organism, e.g. Streptomyces hygroscopicus subsp. ascomyceticus, ATCC 55087, is grown in the presence of proline or its salts as biosynthetic precursors.

EP 0 478 235 A2

Jouve, 18, rue Saint-Denis, 75001 PARIS

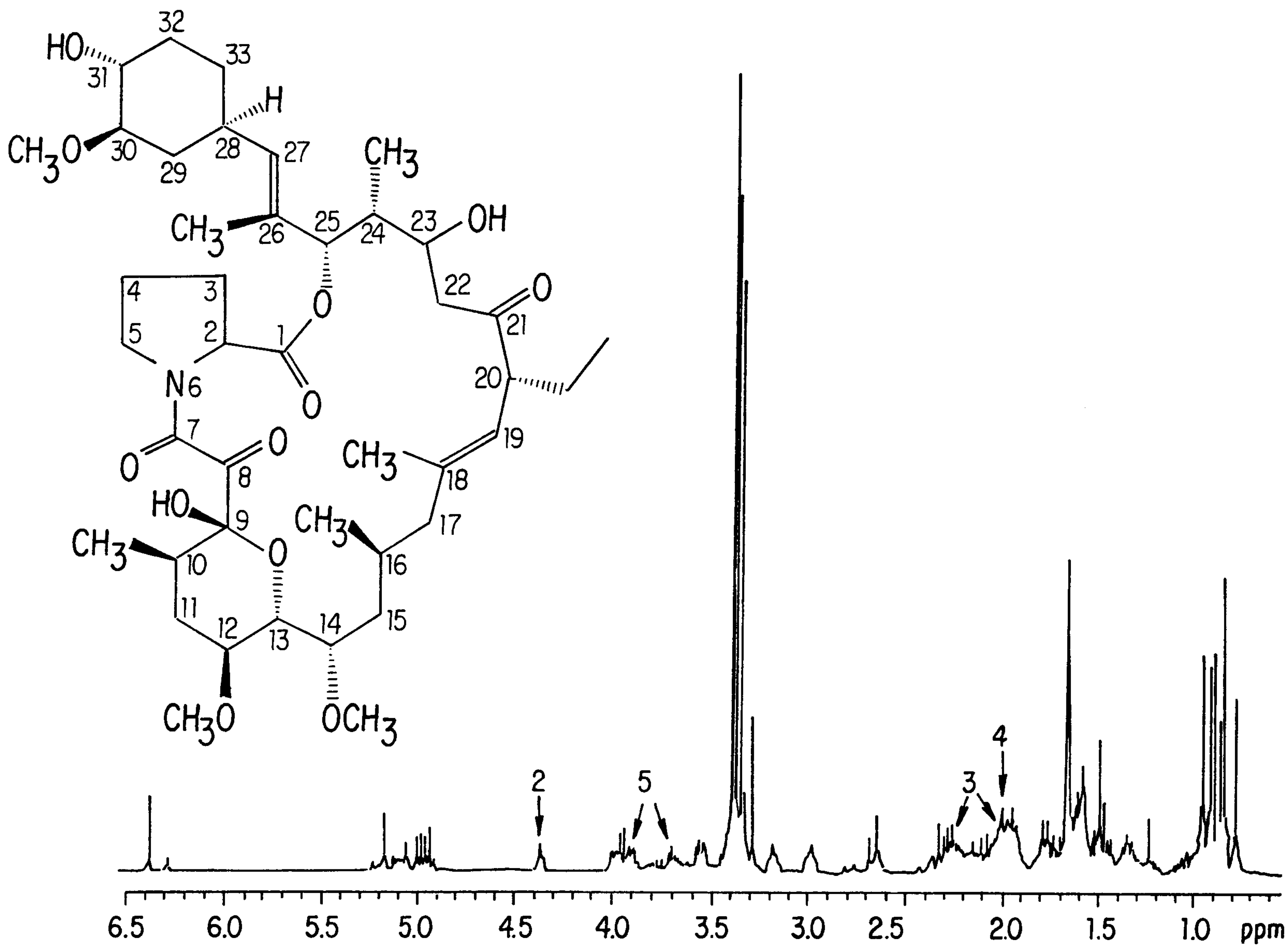


FIG.1

## SUMMARY OF THE INVENTION

The present invention is a fermentation process consisting of the supplemental feeding of a mutant strain (ATCC 55087) of Streptomyces hygroscopicus var. ascomyceticus, with proline or its salts. The mutant strain without biosynthetic direction, produces immunomycin. The novel fermentation process of this invention is referred to as "directed biosynthesis" and is an effective method of producing the new proline analog of FK-520, "prolyl-immunomycin" when the culture is supplemented with proline or its salts.
Immunomycin and its proline analogs are described in the following formula.

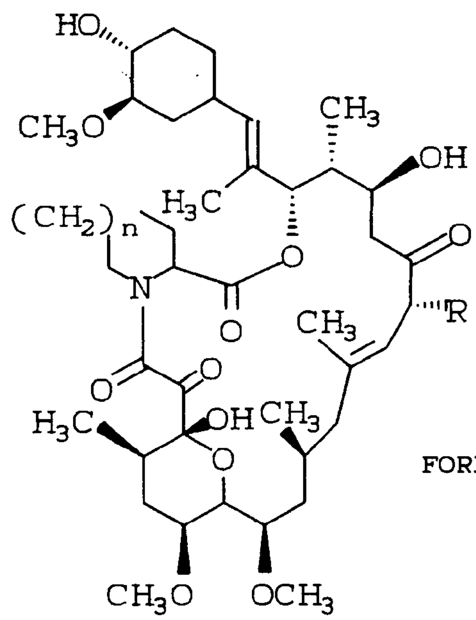

FORMULA I

Immunomycin, $R=CH_2CH_3$, $n=2$

Prolyl-Immunomycin, $R=CH_2CH_3$, $n=1$

The natural biosynthetic pathway utilizes proline or a biologically activated form of proline as a precursor for the proline ring in the synthesis of the proline analog of immunomycin i.e., where n is one in Formula **I**. The organism (ATCC 55087) has incorporated proline, directing the biosynthesis of the proline homolog of immunomycin.

## BACKGROUND OF THE INVENTION

Immunomycin is a fermentation product of the Streptomyces hygroscopicus var. ascomceticus culture (ATCC 14891). Immunomycin has activity superior to cyclosporin, which at the present time is the sole FDA approved anti-rejection drug used in the field of organ transplants. The immunosuppressant activity is also useful as a method of treatment of autoimmune diseases, as well as infectious diseases. The serious side effects associated with cyclosporin treatment include kidney failure, liver damage, and ulcers, and has prompted the development of alternate treatments. The current goal in this field of research is to develop a safer and a more effective method of treatment.

A mutant strain (ATCC 55087) of Streptomyces hygroscopicus var. ascomyceticus (ATCC No. 14891) surprisingly has now been shown to produce the new proline analog of immunomycin by a directed biosynthesis.

## BRIEF DESCRIPTION OF THE FIGURE

Figure I depicts the assigned molecular structure for "prolyl-immunomycin" and its proton NMR at 200 $MH_z$ in $CDCl_3$.

DETAILED DECRIPTION OF THE INVENTION AN PREFERRED EMBODIMENTS

The novel process of this invention comprises the fermentative production of a new compound of Formula I:

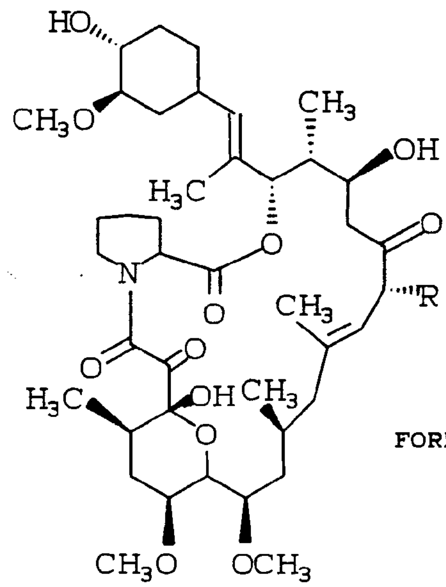

FORMULA I

wherein R is ethyl,

by fermentation of Streptomyces hygroscopicus var. ascomyceticus (ATCC 14891), or a mutant strain thereof, e.g. ATCC 55087, in a nutrient medium, in the presence of proline or a salt thereof, as a biosynthetic directing component.

The following is a general description of Streptomyces hygroscopic subsp. ascomyceticus strain MA6678 (ATCC 55087) and the parent culture MA6475 (ATCC 14891). Observations of growth, general cultural characteristics and carbon source utilization were made in accordance with the methods of Shirling and Gottleib (Internat. J. System. Bacteriol. 16:313-340). Chemical composition of the cells was determined using the methods of Lechevalier and Lechevalier (in Actinomycete Taxonomy, A. Kietz and D. W. Thayer, Ed. Society for Industrial Microbiology, 1980). Coloration of the culture was determined by comparison with color standards contained in the Inter-Society Color Council-National Bureau of Standards Centroid Color Charts (U.S. Dept. of Commerce National Bureau of Standards supplement to NBS Circular 553, 1985.)

Source - Strain MA6678 was derived from MA6475 (Streptomyces hygroscopicus subsp. ascomyceticus ATCC 14891).

Analysis of Cell Wall Composition - The peptidoglycan of strains MA6678 and MA6475 both contain L-diaminopimelic acid.

General growth characteristics - Both cultures are found to grow well on Yeast Malt Extract, Glycerol Asparagine, Inorganic Salts-Starch, Oatmeal, and Trypticase Soy agars. Growth occurs at 27° and 37°C. Both cultures also grow well in liquid media such as Yeast Dextrose broth.

Colony morphology MA6678 - (on Yeast Malt Extract Agar) Substrate mycelium is brilliant yellow (83 brill Y &) and colonies are opaque, raised, erose and rubbery. The colony surface is powdery to rough textured. Aerial mycelia appear after 7 days incubation and are white in color (263 White). Spore mass, when present, in white (263 White) and turns to black (267 Black) upon prolonged incubation. MA6475 - Substrate mycelium is medium yellow (87 m.Y) and colonies are opaque, raised erose and rubbery. The colony surface is powdery. Aerial mycelia appear by 14 d and are medium gray (265 med Gray). Spore mass, when present is medium gray (265 med Gray) and turns black (267 Black) upon prolonged incubation.

Micromorphology MA6678 and MA6475 - Aerial mycelium (0.57 - 0.76 μm dia.) arises from a substrate mycelium and is branched and flexous. In mature cultures, the aerial mycelium commonly terminates in spores borne predominantly in spiral chains. Spore mass tends to coalesce into an amorphous mass by 21d.

Miscellaneous physiological reactions MA6678 - Culture does not produce melanoid pigments in tryptone yeast extract broth, starch is hydrolyzed, yellow, non-pH dependant diffusible pigment produced in Pridham-Gottlieb basal medium supplemented with 1% cellobiose, D-fructose or D-mannose. Carbon source utilization pattern is as follows: good utilization of cellobiose, D-fructose, α-D-lactose, β-D-lactose, D-maltose, D-mannitol,

D-mannose, or L-rhamnose; poor utilization of L-arabinose; no utilization of D-arabinose, inositol, D-raffinose, sucrose, D-xylose, or L-xylose.

MA6475 - Culture does not produce melanoid pigments in tryptone yeast extract broth, starch is hydrolyzed. Carbon source utilization pattern is as follows: good utilization of cellobiose, D-fructose, α-D-lactose, β-D-lactose, D-maltose, D-mannitol, D-mannose, L-rhamnose or D-xylose; poor utilization of L-arabinose or inositol; no utilization of D-arabinose, D-raffinose, sucrose, or L-xylose.

Diagnosis - The chemotaxonomic and morphological characteristics of strain MA6678 compare favorably with those of the parent strain MA6475. Phenotypic characteristics which serve to differentiate the MA6678 from the parent strain include: the production of a soluble yellow pigment on some media and the failure to utilize inositol or D-xylose as a sole carbon source. It should be pointed out that the specific epithet Streptomyces hygroscopicus (and hence all subspecies) is currently considered to be a subjective synonym of Streptomyces violaceusniger (Bergey's Manual of Systematic Bacteriology, Vol. 4, 1989). Comparative studies of MA6475 and MA6678 (as well as many other strains of Streptomyces hygroscopicus) with the type strain of Streptomyces violaceusniger are underway to better clarify this group. While MA6678 can be considered a strain of Streptomyces hygroscopicus subsp. ascomyceticus for patent purposes, that name is no longer considered valid for scientific purposes.

Carbohydrate utilization pattern of
Streptomyces hygroscopicus subsp. ascomyceticus
strains MA6475 and MA6678 at 21 days

| Carbon Source | Utilization by MA6475 | Utilization by MA6678 |
|---|---|---|
| D-arabinose | 0 | 0 |
| L-arabinose | 1 | 1 |
| cellobiose | 2 | 2 |
| D-fructose | 2 | 2 |
| inisitol | 1 | 0 |
| α-D-lactose | 2 | 2 |
| β-D-lactose | 2 | 2 |
| D-maltose | 2 | 2 |
| D-mannitol | 2 | 2 |
| D-mannose | 2 | 2 |
| D-raffinose | 0 | 0 |
| L-rhamnose | 2 | 2 |
| sucrose | 0 | 0 |
| D-xylose | 2 | 0 |
| L-xylose | 0 | 0 |
| a-D-glucose(control) | 2 | 3 |

3 = good utilization, 2 = moderate utilization,
1 = poor utilization, 0 = no utilization

**Cultural characteristics of MA6475 and MA6678 at 21 days**

| Medium | Amount of Growth | | Aerial Mycelium | | Soluble Pigments | | Substrate Mycelium | |
|---|---|---|---|---|---|---|---|---|
| | MA6475 | MA6678 | MA6475 | MA6678 | MA6475 | MA6678 | MA6475 | MA6678 |
| Yeast Extract Malt Extract | good | good | Medium gray (265 med. Gray) branched, spiral sporophores | White (263 White) branched, spiral sporophores | none noted | none noted | Medium yellow (87 m.Y) | Brilliant yellow (83 brill Y) |
| Glucose Asparagine | good | sparse | Medium gray (265 med. Gray) branched, spiral sporophores | Yellow white (92 y. White) branched, spiral sporophores | none noted | none noted | Pale yellow (89 p.Y) | Light yellow (86 l. Y) |
| Inorganic Salts Starch | good | good | Medium gray (265 med. Gray) branched, spiral sporophores | Yellow white (92 y. White) branched, spiral sporophores | none noted | none noted | Pale yellow (89 p.Y) | Pale yellow (89 p.Y) |
| Oatmeal | good | good | Dark gray (265 d. .Gray) branched, spiral sporophores | White (263 White) branched, spiral sporophores | none noted | none noted | Gray yellow (90 gy.Y) | Pale yellow (89 p.Y) |
| Sigma Water | fair | sparse | Black (267 Black) edges, spiral sporophores | | none noted | none noted | | |
| Czpek | good | good | Light gray (264 l. Gray) branched, spiral sporophores | White (263 White) branched, spiral sporophores | none noted | none noted | Medium gray (265 med Gy) | Yellow white (92 y White) |
| Peptone Iron | good | good | | | melanin negative | melanin negative | | |

The fermentation of ATCC 55087 to produce prolyl-immunomycin is usually conducted at a temperature between about 20°C and 40°C, preferably 25-35°C, for a period of about 24 hours to 100 hours, which may be varied according to fermentation conditions and scales. Preferably, the production cultures are incubated for about 24 hours at 28°C on a rotary shaker operating at 240 rpm, wherein the pH of the fermentation medium is maintained at below 8.0 to harvest. At 24 to 30 hours, the culture is harvested, the cells washed and then resuspended back to the original volume in an incubation medium. In addition to glucose and a buffer, the incubation medium contains proline or a related salt thereof, e.g. hydrochloride, hydrobromide, hydrosulfate, for the purpose of directing the biosynthesis, at a final concentration of the substrate of 10-30 mM. The fermentations are harvested at 48 or 96 hours or 18-42 hours after the addition of the proline.

The conditions for the production of prolyl-immunomycin in a large quantity, preferably employ submerged aerobic culture conditions. For the production in a small quantity, a shaking or surface culture in a flask or bottle is employed. Furthermore, when the growth is carried out in large tanks, it is preferable to use the vegetative form of the organism for inoculation in the production tanks in order to avoid growth lag in the process of production of analogs. Accordingly, it is desirable first to produce a vegetative inoculum of the organism by inoculating a relatively small quantity of culture medium with spores or mycelia of the organism produced in a "slant" and culturing said inoculated medium, also called the "seed medium", and then to transfer the cultured vegetative inoculum aseptically to large tanks. The fermentation medium, in which the vegetative inoculum is produced, is similar to the medium utilized for the production of prolyl-immunomycin and is generally autoclaved to sterilize the medium prior to inoculation. The pH of the medium is generally adjusted to below 8.0 prior to the autoclaving step by suitable addition of an acid or base, preferably in the form of a buffering solution.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium. Aeration may be effected by passing sterile air through the fermentation mixture.

The prolyl-immunomycin can be produced by culturing (fermenting) the immunomycin substance-producing strain in an aqueous nutrient medium containing sources of assimilable carbon and nitrogen, preferably under submerged aerobic conditions (e.g. shaking culture, submerged culture, etc.). The aqueous medium must be maintained below pH 8.0 at the initiation and termination (harvest) of the fermentation process. A higher pH leads to substantial and/or total loss of product. The desired pH may be maintained by the use of a buffer such as morpholinoethanesulfonic acid (MES), morpholino-propanesulfonic acid (MOPS), and the like, or by choice of nutrient materials which inherently possess buffering properties, e.g. KQ and FKSH production media described herein below.

The preferred sources of carbon in the nutrient medium are carbohydrates such as glucose, xylose, galactose, glycerin, starch, dextrin, and the like. Other sources which may be included are maltose, rhamnose, raffinose, arabinose, mannose, salicin, sodium succinate, and the like.

The preferred sources of nitrogen are yeast extract, meat estract, peptone, gluten meal, cottonseed meal, soybean meal and other vegetable meals (partially or totally defatted), casein hydrolysates, soybean hydrolysates and yeast hydrolysates, corn steep liquor, dried yeast, wheat germ, feather meal, peanut powder, distilled solubles, etc., as well as inorganic and organic nitrogen compounds such as ammonium salts (e.g. ammonium nitrate, ammonium sulfate, ammonium phosphate, etc.,), urea, amino acids, and the like.

The carbon and nitrogen sources, though advantageously employed in combination, need not be used in their pure form, because less pure materials which contain traces of growth factors and considerable quantities of mineral nutrients, are also suitable for use. When desired, there may be added to the medium mineral salts such as sodium or calcium carbonate, sodium or potassium phosphate, sodium or potassium chloride, sodium or potassium iodide, manesium salts, copper salts, cobalt salts, and the like. If necessary, especially when the culture medium forms seriously, a defoaming agent, such as liquid paraffin, fatty oil, plant oil, mineral oil or silicone may be added.

The preferred culturing/production medium for carrying out the fermentation is as follows:

The seed medium consists of glucose 2.0% yeast extract 2.0%, Hy-Case SF 2.0%, $KN0_3$ 0.2%, $CaCl_2 \cdot 2H_20$ 0.002%, $ZnS0_4 \cdot 7H_20$ 0.001%, $MnS0_4 \cdot H_20$ 0.0005%, $FeS0_4 \cdot 7H_20$ 0.0025%, $MgS0_4 \cdot 7H_20$ 0.05%, NaC1 0.05%, and distilled $H_20$ 1.0 liter. The pH of the medium is adjusted to 7.0 with NaOH before autoclaving. Cultures are shaken at 28°C and 200 rpm for 42 hours.

Production cultures are initiated by adding 1.0 ml of seed culture to 30 ml of production medium in a 250 ml nonbaffled flask, incubated at 28°C, and shaken at 240 rpm. Production medium consists of glucose 2.2%, glycerol 2.5%, corn steep liquor 1.0%, yeast extract 1.5%, $CaC0_3$ 0.025, lactic acid 0.2%, L-tyrosine 0.4%, morpholinopropanesulfonic acid 1.0%, and distilled water 1.0 liter.

Cultures are harvested at the beginning of product formation (24-30 hours), centrifuged at 2000 rpm, washed twice with a solution containing glucose at 0.25% and MOPS at 0.25%. The cells are resuspended

back to the original volume in an incubation buffer containing glucose at 1.0%, MOPS at 0.25%, and L-proline at 0.35% (30 mM). A volume of 30 ml resuspended cells are placed in a non-baffled shake flask (or 2.5 ml in a 25 x 150 mm tube) and shaken at 27°C and 220 rpm for 18-24 hours.

The prolyl-immunomycin produced can be recovered from the culture medium by conventional means which are commonly used for the recovery of other known biologically active substances. The prolyl-immunomycin is found in the cultured mycelium and filtrate, and accordingly can be isolated and purified from the mycelium and the filtrate, which are obtained by filtering or centrifuging the cultured broth, by a conventional method such as concentration under reduced pressure, lyophilization, extraction with a conventional solvent, such as methanol and the like, pH adjustment, treatment with a conventional resin (e.g. anion or cation exchange resin, non-ionic adsorption resin, etc.), treatment with a conventional adsorbent (e.g. activated charcoal, silicic acid, silica gel, cellulose, alumina, etc.), crystallization, recrystallization, and the like. A preferred method is solvent extraction, particularly using methanol.

The preferred strain is a mutant strain of Streptomyces hygroscopicus var. ascomyceticus ATCC 55087.

The broth from the fermentation exhibits positive immunosuppressive activity by the "T-cell proliferation assay" and possesses utility on this basis.

The prolyl-immunomycin obtained according to the fermentation processes as explained above can be isolated and purified in a conventional manner, for example, extraction, precipitation, fractional crystallization, recrystallization, chromatography, and the like. They can be used as immunosupressants according to the procedures known in the art.

Suitable salts of these homologs may include pharmaceutically acceptable salts, such as basic salts, for example, alkali metal salt (e.g. sodium salt, potassium salt, etc.), alkaline earth metal salt (e.g. calcium salt, magnesium salt, etc.), ammonium salt, amine salt (e.g. triethylamine salt, N-benzyl-N-methylamine salt, etc.) and other conventional organic salts. It is to be noted that in the aforementioned fermentation reactions and the post-treatment of the fermentation mixture therein, the geometic isomer and/or stereoisomer(s) of analogs of immunomycin due to asymmetric carbon atom(s) or double bond(s) of the compound may occasionally be transformed into the other isomer(s), and such isomers are also included within the scope of the present invention.

The prolyl-immunomycin of the present invention, possesses pharmacological activity such as immunosuppressive activity, antimicrobial activity, and the like, and therefore are useful for the treatment and prevention of the transplantation rejection of organs or tissues such as heart, kidney, liver, medulla ossium, skin, etc., graft-versus-host diseases by medulla ossium transplantation, autoimmune diseases such as rheumatoid arthritis, systemic lupus erythematosus, Hashimoto's thyroiditis, multiple sclerosis, myasthenia gravis, type I diabetes, uveitis, etc., infectious diseases caused by pathogenic microorganisms, and the like.

The pharmaceutical composition of this invention can be used in the form of a pharmaceutical preparation, for example, in solid, semisolid or liquid form, which contains the prolyl-immunomycin of the present invention, as an active ingredient, in admixture with an organic or inorganic carrier or excipient suitable for external, enteral or parenteral applications. The active ingredient may be compounded, for example, with the usual nontoxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an amount sufficient to produce the desired effect upon the process or condition of diseases.

For applying this composition to a human, preferred application is by parenteral or enteral administration. While the dosage of therapeutically effective amount of the prolyl-immunomycin analog varies from, and also depends upon the age and condition of each individual patient to be treated, a daily dose (calculated on the basis of a 70 kg man) of about 0.01-1000 mg, preferably 0.1-500 mg and more preferably 0.5-100 mg, of the active ingredient is generally given for treating diseases, and an average single dose of about 0.5 mg, 1 mg, 5 mg, 10 mg, 50 mg, 100 mg, 250 mg and 500 mg is generally administered.

The following examples are given for the purpose of illustrating the present invention and should not be construed as being limitations on the scope or spirit of the instant invention.

EXAMPLE 1

MICROORGANISM AND CULTURE CONDITIONS

Streptomyces hygroscopicus var. ascomyceticus was originally obtained from the American Type Culture

Collection (No. 14891). It was deposited in the Merck culture collection and designated MA6475. A strain of MA6475 which produces increased quantities of immunosuppressants FK-520 and FK-523 was isolated from a mutated population of MA6475 and designated MA6678, ATCC No. 55087.

EXAMPLE 2

FERMENTATION

Cultivation was begun by adding 1.0 ml of frozen vegetative cells, preserved in 10% glycerol, to 50 ml of seed medium in a 250 ml baffled eyrlenmeyer. The seed medium consisted of glucose 2.0% yeast extract 2.0%, Hy-Case SF 2.0%, $KN0_3$ 0.2%, $CaCl_2 \bullet 2H_20$ 0.002%, $ZnS0_4 \bullet 7H_20$ 0.001%, $MnS0_4 \bullet H_20$ 0.0005%, $FeS0_4 \bullet 7H_20$ 0.0025%, $MgS0_4 \bullet 7H_20$ 0.05%, NaC1 0.05%, and distilled $H_20$ 1.0 liter. The pH of the medium was adjusted to 7.0 with Na0H before autoclaving. Cultures were shaken at 28°C and 220 rpm for 42 hours. Thirty-five production cultures were initiated by adding 1.0 ml of seed culture to 30 ml of production medium in each 250 ml non-baffled flask, incubated at 28°C, and shaken at 240 rpm. Production medium consisted of glucose 2.2%, glycerol 2.5%, corn steep liquor 1.0%, yeast extract 1.5%, $CaC0_3$ 0.025, lactic acid 0.2%, L-tyrosine 0.4%, morpholinopropane-sulfonic acid 1.0%, and distilled water 1.0 liter. The pH of the medium was adjusted to 6.8 with NaOH before autoclaving. Cultures were harvested at the beginning of product formation (21 hours), centrifuged at 2000 rpm, washed twice with a solution containing glucose at 0.25% and MOPS at 0.25%. The cells were resuspended back to the original volume in incubation buffer containing glucose at 1.0%, MOPS at 0.25%, and L-proline at 0.35% (30 mM). A volume of 30 ml resuspended cells was placed in each of 32 non-baffled shake flasks, and shaken at 28°C and 220 rpm. At 23 hours, 7.5 ml of a sterile solution containing glucose at 40.0 g/l and MOPS at 10 g/l, pH 6.8 was added to each flask. The flasks were incubated at 28°C and 220 rpm for an additional 20 hours and then pooled. An HPLC assay of the pooled broth indicated a titer of 74.2 ug/ml of immunomycin, 23.0 ug/ml of the prolyl-immunomycin proline analogue, and 11.5 ug/ml of FK-523. The pooled broth was extracted with an equal volume of ethyl acetate and the extract dried under vacuum. The dried residue was assayed by HPLC and contained 77.1 mg immunomycin, 27.9 mg of prolyl-immunomycin, and 10.8 mg of FK-523. Separation of prolyl-immunomycin from immunomycin and was accomplished by three preparative runs through a Rainin preparative HPLC column. This afforded 7.2 mg of 98.6% pure prolyl-immunomycin.

EXAMPLE 3

ISOLATION OF PROLYL-IMMUNOMYCIN

The whole broth was centrifuged and the mycelium extracted with methanol (1:1, W/V). The methanol was removed using a rotary evaporator and the resulting aqueous solution was extracted with an equal volume of ethyl acetate. The ethyl acetate extract was dried over anhydrous sodium sulfate, filtered and dried under vacuum to a solid residue.

CHROMATOGRAPHY

Separation of analogues in the presence of FK-520 was accomplished by chromatographing the material once or repeatedly on a semi-preparative Rainin Dynamax HPLC column. Titers of immunomycin and its analogs were determined by analytical HPLC using a Whatman Partisil-5 C8 column (4.6 mm x 25 mm) operated at 60°C and eluted isocratically with a mobile phase consisting of acetonitrile/ 0.1% $H_3P0_4$ (60:40) run at 1.0 ml/minute. Immunomycin and prolyl-immunomycin were detected at 205 nm. Preparative HPLC was accomplished using a Rainin Dynamax-60A C18 column (21.4mm x 25cm) operated at 50°C and eluted isocratically with acetonitrile/ 0.1% $H_3P0_4$ (55:45) run at 10.0 ml/minute. Detection was at 205 nm.

EXAMPLE 4

FRACTIONATION FOR IL-2 ASSAY

Biological activity of peaks observed in the HPLC profiles were determined by collecting 0.5 minute fractions of a sample run on the analytical HPLC employing a mobile phase of acetonitrile/ 0.1% $H_3P0_4$ (55:45) run at 1.0 ml/min. The pH of each sample was adjusted by adding 25 μl of a 0.2 M solution of morpholinoethanesulfonic acid (MES, pH 7.4) to each 1.0 ml fraction. Fractions were then evaporated to dryness using a Speed Vac Concentrator (Savant) and redissolved in ethanol for I1-2 assay.

EXAMPLE 5

T-CELL PROLIFERATION ASSAY

1. Sample Preparation

Purified compound, or fractions, prepared by HPLC, was dissolved in absolute ethanol at a final concentration of 100 μg/ml.

2. Assay

Spleens from C57B1/6 mice were taken under sterile conditions and gently dissociated in ice-cold RPMI 1640 culture medium (GIBCO, Grand Island, N. Y.) supplemented with 10% heat-inactivated fetal calf serum (GIBCO). Cells were pelleted by centrifugation at 1500 rpm for 8 minutes. Contaminating red cells were removed by treating the pellet with ammonium chloride lysing buffer (GIBCO) for 2 minutes at 4°C. Cold medium was added and cells were again centrifuged at 1500 rpm for 8 minutes. T lymphocytes were then isolated by separation of the cell suspension on nylon wool columns as follows: Nylon wool columns were prepared by packing approximately 4 grams of washed and dried nylon wool into 20 ml plastic syringes. The columns were sterilized by autoclaving at 250°F for 30 minutes. Nylon wool columns were wetted with warm (37°C) culture medium and rinsed with the same medium. Washed spleen cells resuspended in warm medium were slowly applied to the nylon wool. The columns were then incubated in an upright position at 37°C for 1 hour. Non-adherent T lymphocytes were eluted from the columns with warm culture medium and the cell suspensions were spun as above.

Purified T lymphocytes were resuspended at 2.5 x $10^{-5}$ cells/ml in complete culture medium composed of RPMI 1640 medium with 10% heat-inactivated fetal calf serum, 100 mM glutamine, 1mM sodium pyruvate, 2 x $10^{-5}$M 2-mercaptoethanol and 50 μg/ml gentamycin. Ionomycin was added at 250 ng/ml and PMA at 10 ng/ml. The cell suspension was immediately distributed into 96 well flat-bottom microculture plates (Costar) at 200 μl/well. The control, being the medium without test drug, and various below-indicated dilutions of the sample (above-described purified prolylimmunomycin) to be tested were then added in triplicate wells at 20 μl/well. FK-520 was used as a standard. The culture plates were then incubated at 37°C in a humidified atmosphere of 5% $CO_2$-95% air for 44 hours. The proliferation of T lymphocytes was assessed by measurement of tritiated thymidine incorporation. After 44 hours of culturing, the cells were pulse-labelled with 2 μCi/well of tritiated thymidine (NEN, Cambridge, MA). After another 4 hours of incubation, cultures were harvested on glass fiber filters using a multiple sample harvester. Radioactivity of filter discs corresponding to individual wells as measured by standard liquid scintillation counting methods (Betacounter). Mean counts per minute of replicate wells were calculated and the results expressed as percent inhibition of tritiated thymidine uptake (profileration) as follows:

$$\text{Inhibition} = 100 - \frac{\text{Mean cpm sample tested X 100.}}{\text{Mean cpm control medium}}$$

The results of % inhibition at various concentrations of FK-506 are presented in Table 1:

TABLE 1

Inhibition of T Cell Proliferation by Prolyl-Immunomycin (P-I)

| Concentration of P-I (ng/ml) | % Inhibition |
|---|---|
| 62.0 | 97.8 |
| 31.0 | 96.3 |
| 15.5 | 94.3 |
| 7.8 | 84.8 |
| 3.9 | 47.0 |
| 1.9 | 8.2 |

Notes:

1. Mouse T cell cultures were pulsed with 3H-thymidine for 4 hours prior to harvesting at 48 hours.
2. Standard L-683,590 (FK-520) (4 ng/ml) gave 99% inhibition.
3. The mean IC50 for "P-I" was determined to be 5.9 ± 1.6 ng/ml (7.6 ± 2.0 nM) in 6 independent experiments.
4. The inhibition of T cell proliferation by "P-I" was reversed by the addition of 100 units/ml of recombinant human IL-2 at the initiation of culture.

EXAMPLE 6

MASS SPECTROMETRY

A sample from Example 3, containing pure "prolyl-immunomycin" was analyzed by FAB mass spectrometry using a MAT-731 mass spectrometer operated at 8 kv. The matrix employed was a 5:1 mixture of dithiothreitol/dithioerythritol which contained a trace amount of lithium acetate. The sample was ionized using a xenon fab gun at 8 kv. A pseudo-molecular ion was observed at m/z = 784 (M + Li) corresponding to a nominal molecular weight of 777 amu. A sodium adduct ion (M + Na + matrix) was observed at m/z = 800 (M + Na). The mass spectrum was in agreement with the assigned molecular structure as depicted in Figure 1.

NUCLEAR MAGNETIC RESONANCE

The proton spectrum of "prolyl-immunomycin" measured at 400 $MH_z$ in $CDCl_3$, given in Figure 1, strongly supports the assigned molecular structure of "prolyl-immunomycin" as also given in Figure 1.

## Claims

1. A process for the preparation of a compound of Formula I

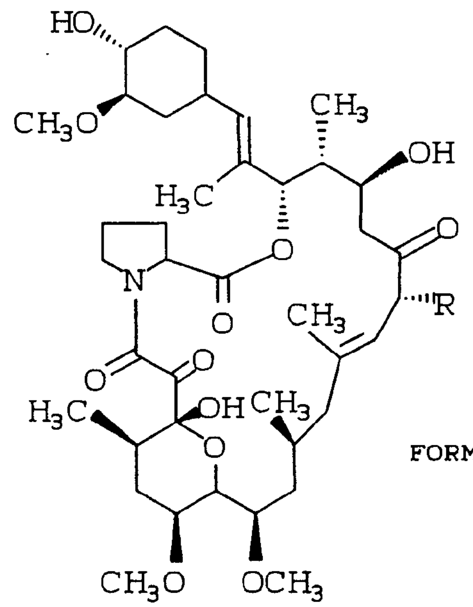

FORMULA I

wherein R is ethyl which comprises fermenting an immunomycin producing strain of Streptomyces in aqueous nutrient medium containing an assimilable source of carbon, an assimilable source of nitrogen and inorganic salts under aerobic conditions in the presence of proline, or its salts.

2. The process of Claim 1 wherein the strain is Streptomyces hygroscopicus var. ascomyceticus, ATCC No. 14891.

3. The process of Claim 1 wherein the strain is a mutant strain of Streptomyces hygroscopicus var. ascomyceticus (ATTC 14891).

4. The process of Claim 3, wherein the directed biosynthesis employs the mutant strain of Streptomyces hygroscopicus var. ascomyceticus identified as ATCC 55087, (Merck No. MA6678).

5. The process of Claim 4, wherein the fermentation is conducted at a temperature between 24 and 30°C and a pH range from about 6.0 to 7.5, with agitation supplied by a rotary shaker operating between 150 and 300 rpm.

6. The process of Claim 4, wherein the nutrient medium contains about 0.5 to 5.0% by weight of carbon source and about 0.2 to 6.0% by weight of nitrogen.

7. The process of Claim 5, wherein the fermentation is carried out for 1 to 8 days.

8. A process for the preparation of a seed culture for fermentative production of the compound of Formula I which comprises a fermentation of the mutant strain ATCC 55087, Merck No. (MA6678): 1) using 1.0 ml frozen vegetative cells preserved in 10% glycerol to 50 ml of a seed medium which consists of: glucose 2.0%,yeast extract 2.0%, Hy-Case SF 2.0%, $KN0_3$ 0.2%, $CaCl_2{\cdot}2H_20$ 0.002%, $ZnS0_4{\cdot}7H_20$ 0.001%, $MnS0_4{\cdot}H_20$ 0.0005%, $FeS0_4{\cdot}7H_20$ 0.0025%, $MgS0_4{\cdot}7H_20$ 0.05%, NaCl 0.05%, and 1.0 L distilled $H_20$; 2) adjusting the pH of the medium to 7.0 with NaOH; 3) autoclaving; and 4) shaking the tubes at 230 rpm for 42 hours at 27°C.

9. The process for the preparation of a compound of Formula I which comprises 1) the mixing of 0.5 ml of the seed culture of Claim 8 with 15 ml of production medium consisting of: glucose 2.2%, glycerol 2.5%, corn steep liquor 1.0%, yeast extract 1.5%, $CaC0_3$ 0.025, lactic acid 0.2%, L-tyrosine 0.4%, morpholinopropanesulfonic acid 1.0%, and 1.0L distilled water; 2) incubating at 27°C with agitation at 240 rpm; 3) adjusting pH to 6.8; 4) autoclaving; 5) adding proline, or salt thereof, during the first 54 hours; and 6) harvesting at 3-4 days.

**10.** A compound of Formula I

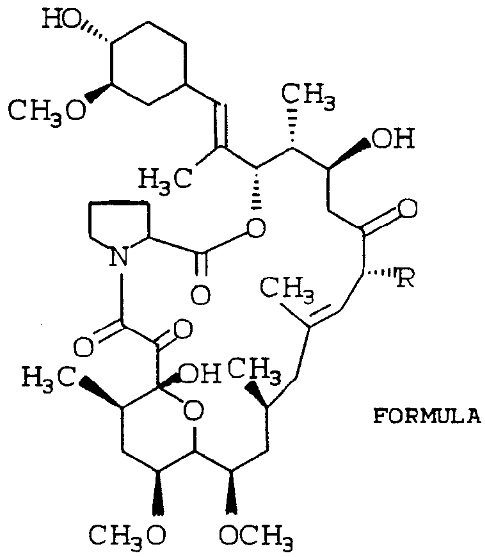

FORMULA I

wherein R is ethyl

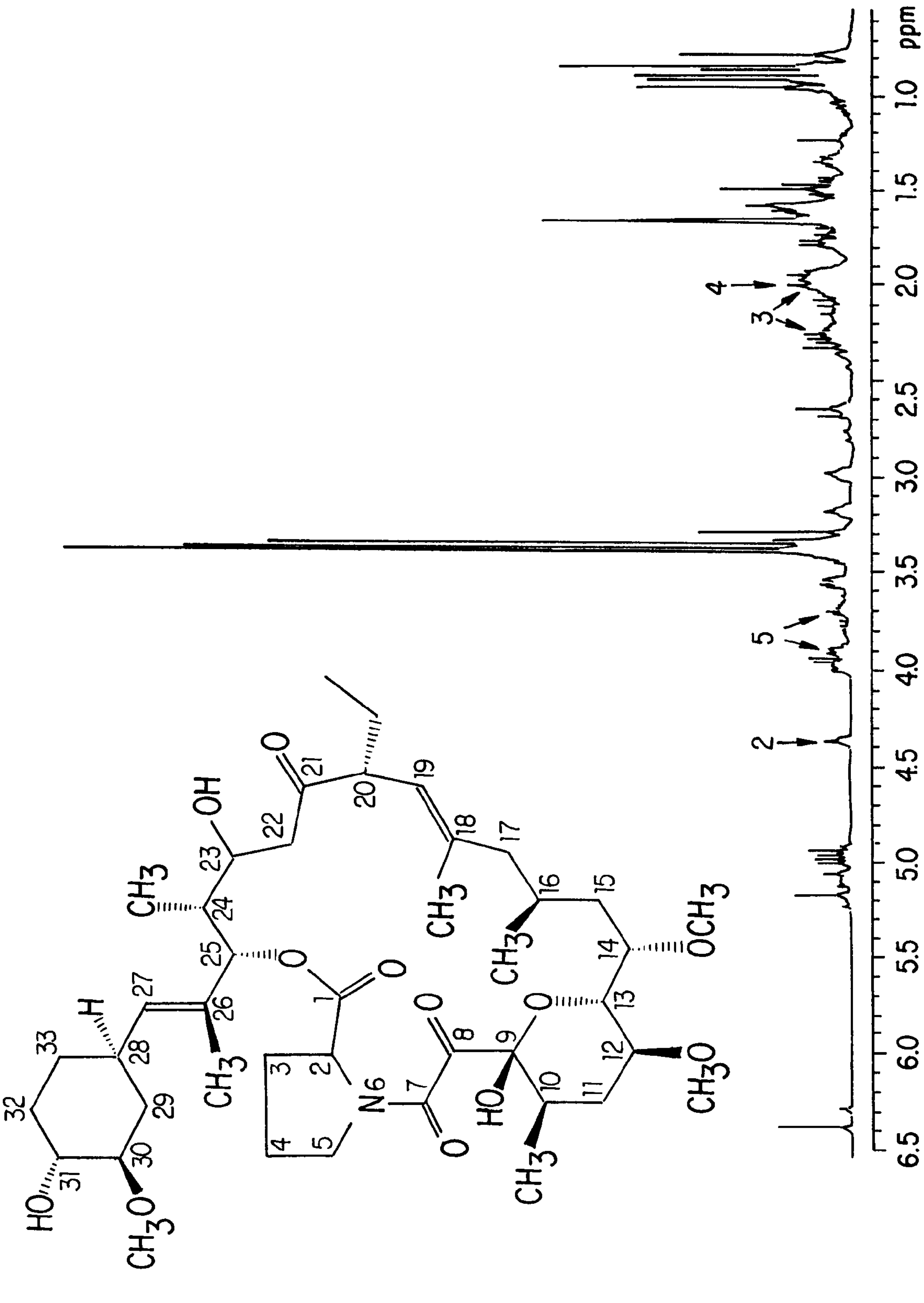


FIG. 1